# EUROPÄISCHE PATENTANMELDUNG

(11) **EP 0 788 804 A2**
(43) Veröffentlichungstag der Anmeldung: **13.08.1997**
(21) Anmeldenummer: 97100173.0
(22) Anmeldetag: 08.01.1997
(51) Int. Cl.: A61M 5/24

(54) **Applikationsvorrichting für medizinische Flüssigkeiten**

(30) Priorität: 08.02.1996 DE 29602173 U
(71) Anmelder: B. BRAUN MELSUNGEN AG, 34212 Melsungen (DE)
(72) Erfinder: Ibarra, Miquel, 0804 Barcelona (ES); Fuchs, Jürgen, 34308 Bad Emstal (DE); Weissmann, Wilm, 34212 Melsungen (DE); Brungs, Heribert, Dr., 34225 Baunatal (DE)
(74) Vertreter: Selting, Günther, Dipl.-Ing.

(57) **Zusammenfassung**

Die Applikationsvorrichtung weist einen geschlossenen, mindestens teilweise flexiblen einstückigen Behälter (10a) auf, der ein zum Freilegen einer Behälteröffnung (20) abtrennbares Teil aufweist. Um den Behälterinhalt unmittelbar aus dem Behälter an den Zielort zu übertragen, ist eine Übertragungsvorrichtung aus einem Konnektor (22b) vorgesehen, welcher abdichtend an der freigelegten Behälteröffnung (20) anbringbar ist. Der Konnektor ist mit einer Kanüle versehen oder mit einer Kanüle verbindbar. Mit der Kanüle kann durch einen Einstich in den Zielort der Behälterinhalt durch Zusammendrücken des Behälters unmittelbar an den Zielort übertragen werden.

## Beschreibung

Die Erfindung betrifft eine Applikationsvorrichtung für medizinische Flüssigkeiten, wie z.B. Injektionslösungen.

Injektionslösungen werden bis zu ihrer Verwendung in Ampullen oder Vials aufbewahrt. Neben Glasgebinden haben Kunststoffampullen eine immer größere Bedeutung erlangt. Derartige Ampullen sind einstückige Behälter, die vollständig geschlossen sind und ein zum Freilegen einer Behälteröffnung abtrennbares Teil aufweisen. Nach dem Abtrennen dieses Teils wird die Injektionslösung in der Regel in eine Spritze aufgezogen und anschließend mit einer an der Spritze befestigten Kanüle an den Zielort übertragen. Zielorte sind bei medizinischen Lösungen:
1. ein Behältnis, z.B. ein Vial, in dem sich ein Inhalt befindet, der verdünnt, aufgelöst, emulgiert oder suspendiert werden muß,
2. ein Infusionslösungsbehältnis, aus dem die Lösung später in verdünnter Form in den Körper eines Patienten übertragen wird,
3. der Körper eines Patienten.

Die Spritze wird hierbei bisher als unerläßlich angesehen, denn sie dient als Transportvehikel zum Aufziehen und Überführen der Lösung an den Zielort, als Dosiereinrichtung zum Abmessen eines definierten Volumens und als Servereinheit für die Kanüle zur Übertragung der Lösung in geschlossene aseptische Systeme.

Der Erfindung liegt die Aufgabe zugrunde, eine vereinfachte Applikationsvorrichtung zu schaffen, die es ermöglicht, die Flüssigkeit direkt aus dem Behälter an den Zielort zu überführen.

Die Lösung dieser Aufgabe erfolgt erfindungsgemäß mit den im Patentanspruch 1 angegebenen Merkmalen.

Nach der Erfindung ist ein Konnektor vorgesehen, der unmittelbar an der freigelegten Behälteröffnung anbringbar ist und diese abdichtet. Dieser Konnektor kann eine ihm angeformte Kanüle oder einen Ansatz zur Anbringung einer separaten Kanüle aufweisen. Auf diese Weise entsteht eine Behälter-Kanülen-Einheit, die infolge der mindestens teilweise flexiblen Behälterstruktur zugleich als Spritze verwendbar ist. Die Kanüle dieser Einheit kann unmittelbar in das Zielbehältnis eingestochen werden. Danach wird die Flüssigkeit durch wiederholtes Drücken auf den flexiblen Bereich der Behälterwand und anschließendes Entspannen in Pumpbewegungen in das Zielbehältnis überführt. Während bei der Übertragung mit einer Spritze durch das Zusatzvolumen im Zielbehälter ein Überdruck entsteht, besteht der besondere Vorteil des repetierenden Pumpens darin, daß zwischen beiden Behältern ein Druckausgleich stattfindet. Das besonders Berufsanfängern bekannte Phänomen, daß sich bereits bei mittleren Volumina, die zu übertragen sind, aufgrund des gebildeten Überdrucks im Zielbehältnis Kanüle und Spritzenkonus trennen und die gepreßte Flüssigkeit aus dem System spritzt, wird hierbei vermieden. Da die Übertragung von Luft bei halbstarren Behältnissen nicht sicher ausgeschlossen werden kann, ist die Erfindung für die direkte Übertragung in das Blutgefäßsystem eines Patienten als Zielort wegen der damit verbundenen Emboliegefahr insbesondere mit Behältern in Form kollabierbarer Beutel praktikabel.

Die repetierende Pumpfunktion wird am günstigsten mit im Querschnitt runden oder ovalen Behältern realisiert. Durch die repetierende Pumpfunktion werden jeweils Teilmengen der gesamten Injektionslösung übertragen. Nach dem Entspannen des Behälters ist die Höhe des Flüssigkeitsspiegels der verbleibenden Lösung im Behälter proportional dem Restvolumen. Durch Anbringung einer Skalierung am Behälter kann die Überführung der Lösung mit hinreichender Dosiergenauigkeit auf eine definierte Teilmenge begrenzt werden.

Die erfindungsgemäße Applikationsvorrichtung hat den Vorteil, daß eine separate Spritze nicht erforderlich ist. Vielmehr kann die Lösung aus dem Behälter direkt an ihren Zielort überführt werden. Es besteht sogar die Möglichkeit der retrograden Aufnahme der fertigen Flüssigkeit (z.B. ein aufgelöstes Arzneistofflyophylisat aus einem Vial), um sie danach z.B. in ein Infusionsbehältnis einzudosieren. Für den Anwender ergeben sich daraus folgende Vorteile:
- schnelleres und einfacheres Arbeiten, da das Auspacken und Aufziehen einer Spritze nicht erforderlich sind,
- hygienischeres Arbeiten, da das System überdruckfrei und nahezu geschlossen ist, unsterile Luft von der Lösung ferngehalten wird und keine Fremdstoffe, wie Kolbengleitmittel und andere Spritzenstoffe, in das System eindringen können,
- Umweltschonung bei Herstellung und Entsorgung, da das Hilfsmittel Spritze inkl. Verpackung vollständig eingespart wird,
- Verminderung des technischen Aufwandes und dadurch Kostensenkung.

Die Erfindung ist vorzugsweise mit Behältern realisierbar, die als Kunststoffampullen ausgebildet sind, jedoch auch mit Beuteln. Derartige Behälter haben in der Regel Maßungenauigkeiten bzw. Toleranzen, oder die Trennstelle, an der das Öffnen des Behälters erfolgt, ist häufig nicht scharf begrenzt, so daß mit einem starren spritzgeformten Konnektor nicht immer eine dichte Verbindung erreicht werden kann. Dies beruht zum einen darauf, daß die Behälter nach dem Bottel-Pack-Verfahren in einem Arbeitsgang geformt, befüllt und verschlossen werden und dabei geringe Formengrate zurückbleiben, zum anderen darauf, daß i.d.R. nach der Fertigung eine Autoklavsterilisation erfolgt, deren thermische Belastung die Maßhaltigkeit der Konnexionsstelle beeinflussen kann. Der erfindungsgemäße Konnektor ist so ausgebildet, daß er trotz dieser Ungenauigkeiten die Behälteröffnung wirksam abdichtet.

Weitere Vorteile und Merkmale der Erfindung sind in den Unteransprüchen sowie der nachfolgenden Beschreibung von Ausführungsbeispielen zu entnehmen.

Im folgenden werden Ausführungsbeispiele der Erfindung unter Bezugnahme auf die Zeichnungen näher erläutert.

Es zeigen:
- Fig. 1: eine Ausführungsform des Behälters,
- Fig. 2: das Ansetzen eines mit einer Kanüle versehenen Konnektors mit Außenschneidgewinde an die Behälteröffnung,
- Fig. 3: eine Ausführungsform eines Konnektors mit Außenschneidgewinde, der mit einer separaten Kanüle verbunden wird,
- Fig. 4: eine andere Ausführungsform eines Behälters mit daran befestigtem Konnektor,
- Fig. 5: in vergrößerter Darstellung die Abdichtung der Behälteröffnung durch den Konnektor,
- Fig. 6: eine Ausführungsform eines Konnektors mit elastomerem Dichtungsring und Innenschneidgewinde,
- Fig. 7: eine gegenüber Fig. 4 modifizierte Ausführungsform des Konnektors mit elastischer, ringwulstartiger Arretierung zur Fixierung der aufgesetzten Kanüle,
- Fig. 8: einen Konnektor mit zwei männlichen Luer-Ansätzen und
- Fig. 9: einen Konnektor mit eingesetztem Dichtelement.

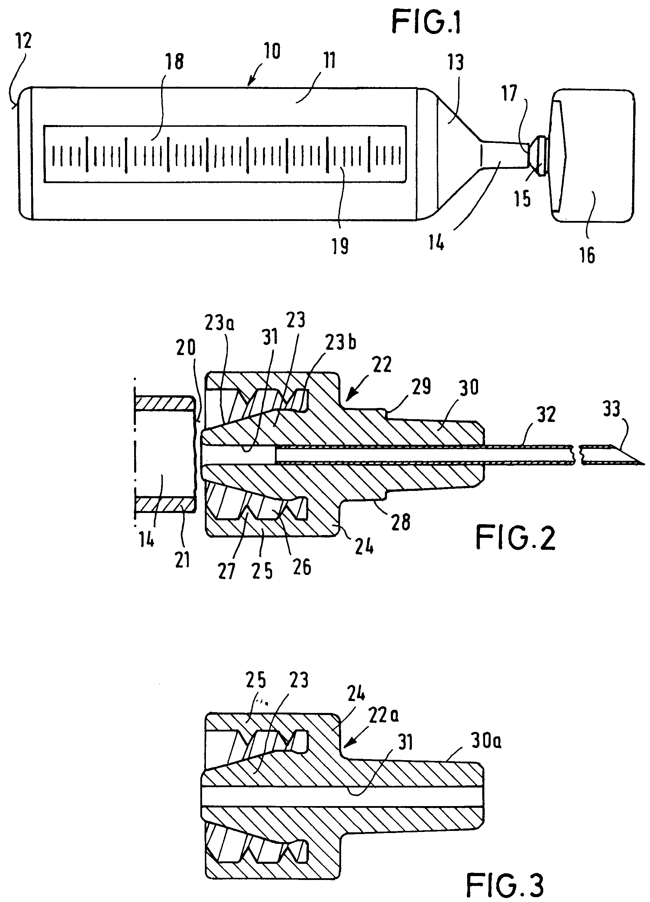

In Fig. 1 ist eine Ausführungsform eines handelsüblichen Behälters 10 dargestellt. Dieser besteht aus einer Ampulle, die einstückig aus Kunststoff hergestellt ist. An einen zylindrischen Behälterkörper 11, der durch eine Bodenwand 12 verschlossen ist, schließt sich ein konischer Behälterhals 13 an, der in einen zylindrischen Kanal 14 übergeht. Der Kanal 14 ist an seinem vorderen Ende mit einem Wandteil 15 verschlossen, an das sich seitlich abstehende Flügel 16 als Drehknebel anschließen. Zwischen der Wand des Kanals 14 und der Wand 15 ist eine ringförmig umlaufende Sollbruchstelle 17 vorgesehen. Durch Angreifen mit der Hand an den Flügeln 16 kann das Wandteil 15 von dem Kanal 14 abgedreht und entfernt werden, so daß dann die Behälteröffnung freiliegt und offen ist. Der Behälter 10 enthält eine medizinische Flüssigkeit, z.B. eine Injektionslösung.

Auf den Behälterkörper 10 ist ein Etikett 18 in Form eines längslaufenden Streifens aufgeklebt. Dieses Etikett trägt eine Graduierung 19, die an dem dem Behälterboden zugewandten Ende den Maximalwert aufweist, also eine sogenannte Überkopfgraduierung. Dadurch kann bei mit der Behälteröffnung nach unten gerichtetem Behälter die noch im Behälter befindliche Flüssigkeitsmenge an der Graduierung 19 abgelesen werden.

Der Behälter ist im Lieferzustand vollständig geschlossen. Er ist einstückig ausgeführt. Dies bedeutet, daß sämtliche Behälterteile miteinander verschweißt oder verklebt oder auf andere Weise bleibend miteinander verbunden sind, und daß keine Teile vorhanden sind, die zerstörungsfrei vom Behälter gelöst werden können.

In Fig. 2 ist die nach dem Abtrennen des Teils 15 freiliegende offene Behälteröffnung 20 dargestellt, die das Ende des Kanals 14 bildet. An der Wand 21, die den Kanal 14 begrenzt, wird der Konnektor 22 befestigt.

Der Konnektor 22 weist gemäß Fig. 2 einen axial abstehenden Ansatz 23 auf, der in den Kanal 14 einführbar ist. Der Ansatz 23 hat einen äußeren konischen Bereich 23a, an dessen dickeres Ende sich ein leicht eingezogener Bereich 23b anschließt, dessen Außendurchmesser etwas größer ist als der Innendurchmesser des Kanals 14. An den Bereich 23b schließt sich ein radialer Ringkragen 24 an, von dem sich eine Muffe 25 nach vorne erstreckt, die etwa bis zum vorderen Ende des Ansatzes 23 reicht. Zwischen dem Ansatz 23 und der Muffe 25 ist ein Ringraum 26 gebildet. An der Innenseite der Muffe 25 verläuft eine schraubenförmige Dichtrippe 27 mit beispielsweise dreieckförmigem Profil und Schneidringfunktion. In den Ringraum 26 kann die ringförmige Wand 21, die den Kanal 14 des Behälters umgibt, vom vorderen Ende her eingeschraubt werden, wobei der konische Bereich 23a in den Kanal 14 eindringt und die Rippe 27 abdichtend gegen die Außenseite der Wand 21 drückt. Eine weitere Abdichtung erfolgt durch den Preßsitz, mit dem die Innenseite der Wand 21 auf dem dicken Bereich 23b des Ansatzes aufsitzt.

Von dem Ringkragen 24 erstreckt sich ein Hals 28 nach hinten, welcher über eine Ringschulter 29 in einen als Konus ausgebildeten Kanülenansatz 30 übergeht.

Durch den gesamten Konnektor 20 verläuft ein Kanal 31, der vom Ende des Ansatzes 23 bis zum Ende des Ansatzes 30 reicht. In diesen Kanal 31 ist eine aus Stahl bestehende Kanüle 32 eingesteckt, die aus dem Ansatz 30 herausragt und an ihrem Ende eine schneidende Spitze 33 aufweist. Die Kanüle 32 ist hohl und in dem Ansatz 22, z.B. durch Kleben, befestigt. Alternativ kann der Konnektor auch einstückig mit angeformter Kanülenspitze aus Kunststoffmaterial gefertigt sein.

Nach dem Abtrennen des Teils 15 vom Behälter 10 wird der Konnektor 22 abdichtend auf die Wand 21 des Kanals 14 aufgeschoben. Dann wird die Kanüle 32 in den Zielort eingestochen. Durch Zusammendrücken des flexiblen Behälters 10 kann dann die Flüssigkeit durch die Kanüle hindurch aus dem Behälter ausgedrückt werden. Zur Verminderung des nicht entnehmbaren Flüssigkeitsvolumens kann der Ansatz 23 im Behälterbereich geschlitzt oder durchbohrt sein, wobei die Schlitze oder Bohrungen zusätzliches Volumen zur Verfügung stellen.

Der Konnektor 22a von Fig. 3 ist in gleicher Weise ausgebildet wie der Konnektor 22 von Fig. 2, jedoch mit der Ausnahme, daß er keine integrierte Kanüle 32 aufweist. Er hat einen männlichen Luer-Konus 30a, auf den der weibliche Luer-Ansatz einer separaten Kanüle aufgesetzt werden kann, wobei dann der Kanülenkanal mit dem Kanal 31 des Konnektors 22a abdichtend verbunden wird.

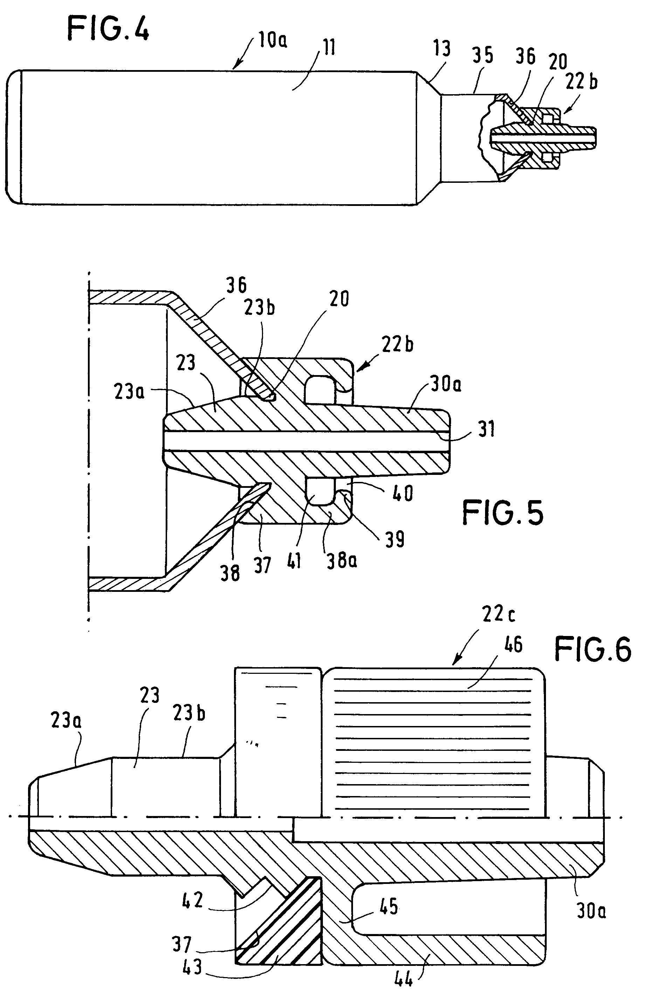

In Fig. 4 ist eine andere Ausführungsform des Behälters 10a dargestellt. Bei diesem Behälter 10a schließt sich an die Ringschulter 13 ein Hals 35 an, der in eine konische Wand 36 übergeht. Die Behälteröffnung 20 befindet sich an dem kleineren Ende der konischen Wand 36, von dem das abtrennbare Teil (in Fig. 4 nicht dargestellt) abgedreht worden ist. In die Behälteröffnung 20 ist der Konnektor 22b eingesteckt.

Der Konnektor besteht vorzugsweise aus Elastomermaterial, das hinreichend weich ist, um eine Abdichtung gegenüber der Behälteröffnung zu bewirken.

Wie aus Fig. 5 hervorgeht, hat der Konnektor 22b ebenfalls einen Ansatz 23, der in gleicher Weise ausgebildet ist wie der Ansatz von Fig. 2, jedoch ist eine den Ansatz übergreifende Muffe nicht vorhanden. Der Konnektor ist mit einem angeformten Dichtkragen 37 versehen, der den dicken Teil 23b des Ansatzes 23 übergreift und eine konische Innenwand 38 aufweist. In die durch die Innenwand 38 und den Teil 23b begrenzte Hinterschneidung wird die Wand 36 des Behälters hineingedrückt, wobei die Behälteröffnung 20 den dicken Teil 23b des Ansatzes 23 umspannt. Dadurch wird der Konnektor 22b an der Behälteröffnung abdichtend festgehalten. Für eine weitere Abdichtung an der Außenfläche der Behälterwand 36 sorgt der Dichtkragen 37.

An seinem kanülenseitigen Ende weist der Konnektor 22b einen Ansatz 30a zum Aufstecken einer Kanüle auf. Dieser Ansatz 23a wird auf einem Teil seiner Länge von einer Muffe 38a übergriffen, die eine elastische Innenrippe 39 aufweist. Zwischen dem Ansatz 30a und der Innenrippe 39 befindet sich ein Ringspalt 40, der in einen von dem Ansatz 30a und der Muffe 38 begrenzten Ringraum 41 einmündet. Die elastische Innenrippe 39 federt beim Aufstecken eines weiblichen Kanülenansatzes auf den Ansatz 30a zurück und rastet hinter dem Wulst des Kanülenansatzes ein. Die Innenrippe 39 bildet also eine Schnappverbindung zum Festhalten des auf den Ansatz 30a aufgeschobenen (nicht dargestellten) Kanülenansatzes.

Auch bei dem Ausführungsbeispiel von Fig. 5 besteht der Konnektor 22b aus einem einstückigen Elastomerteil.

Fig. 6 zeigt einen Konnektor 22c mit einem in die Behälteröffnung einführbaren Ansatz 23, der angrenzend an seinen dickeren Teil 23b mit einem Schneidgewinde 42 versehen ist, welches in die Behälteröffnung eingedreht wird. Über dem Schneidgewinde sitzt ein Dichtungsring 43, der einen die Behälteröffnung übergreifenden Dichtkragen 37 bildet.

Von dem Schneidgewinde 42 erstreckt sich ein Ansatz 30a, auf den ein Kanülenansatz aufgesteckt werden kann. Der Ansatz 30a ist von einer Muffe 44 übergriffen, an deren Stirnwand 45 sich der Dichtungsring 43, der dreieckförmigen Querschnitt hat, abstützt. Die Muffe 44 weist an ihrer Außenseite eine Riffelung 46 auf, um den Konnektor 22c mit der Hand festhalten und führen zu können. Der Konnektor 22c von Fig. 6 besteht aus dem einstückigen Konnektorkörper, der aus Hartkunststoff hergestellt ist, und dem daraufsitzenden Dichtungsring 43. Er ist für die in Fig. 4 dargestellte Behälterart bestimmt.

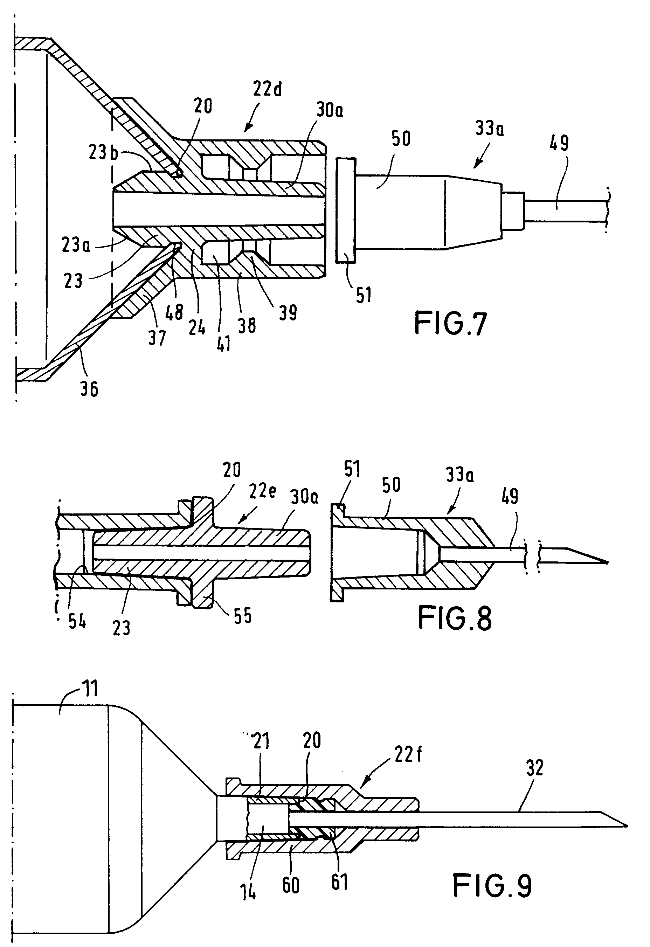

Für diese Behälterart ist auch der Konnektor 22d von Fig. 7 bestimmt, der mit einem Ansatz 23 und einem den Ansatz 23 übergreifenden Dichtkragen 37 ausgestattet ist. Der Dichtkragen 37 besteht hier aus einem kegelstumpfförmigen elastischen Flügel, der von dem Ringkragen 24 trichterförmig abgeht. Zwischen dem dicken Teil 23b des Ansatzes 23 und der Wurzel des Dichtkragens 37 befindet sich am Ansatz 23 eine Nut 48, in der die Behälteröffnung 20 einrastet. Der Dichtkragen 37 drückt dabei von außen gegen die Wand 36 des Behälters.

Über die gesamte Länge des Ansatzes 30a, auf den die Kanüle aufgeschoben wird, erstreckt sich eine Muffe 38, die eine Innenrippe 39 aufweist.

In Fig. 7 ist ferner die Kanüle 33a dargestellt, die mit dem Konnektor 22d verbunden wird. Diese Kanüle 33a weist ein Kanülenrohr 49 auf, an dem ein Kanülenansatz 50 mit Luer-Innenkegel angebracht ist. Der Kanülenansatz 50 hat am Ende einen Wulst 51, der an der nachgiebigen Innenrippe 39 vorbei in den Ringraum 41 des Konnektors 22d eingeschoben und verrastet wird. Der gesamte Konnektor 22d besteht aus Elastomermaterial.

Der Konnektor 22e von Fig. 8 ist die einfachste Form der Erfindung und besteht aus einem Konnektor mit beidseitigem männlichem Luer-Konus. Er wird bei einem Behälter benutzt, der angrenzend an die Behälteröffnung 20 einen Luer-Innenkegel aufweist. Bei dem Behälter ist der Mündungskanal 14 bei der Fertigung mittels Kalibrierdorn exakt ausgeformt und mit einem Konnektor 22e ohne zusätzlich notwendige Dichtelemente mit einer Kanüle mit ebenfalls weiblichem Luer-Ansatz verbindbar.

Der Konnektor 22e ist mit einem als männlicher Luer-Konus ausgebildeten Ansatz 23 versehen, der in den Luer-Konus 54 eingesteckt wird. Am entgegengesetzten Ende weist der Konnektor 22e einen Ansatz 30a zum Aufschieben des Ansatzes 50 einer separaten Kanüle 33a auf. Zwischen den Ansätzen 23 und 30a befindet sich ein radial abstehender Ringkragen 55.

Der Konnektor 22e kann wahlweise aus Elastomermaterial oder aus starrem Kunststoff bestehen.

Der in Fig. 9 dargestellte Konnektor 22f weist einen Ansatz 60 auf, der als (weiblicher) Innenkonus ausgebildet ist und bei dem es sich hier um einen Kanülenansatz der Kanüle 32 handelt. In dem Innenkegel des Ansatzes 60 befindet sich ein elastomeres Dichtelement 61, durch das die Kanüle 32 hindurchführt. Beim Aufsetzen des Ansatzes 60 auf die freigelegte Öffnung des Behälters 11 drückt die den Kanal 14 begrenzende Wand 21, auf die der Ansatz 60 aufgeschoben wird, axial gegen das Dichtelement 61. Das Ende der Wand 21 wird dadurch von dem Dichtelement 61 abgedichtet. Auf diese Weise wird ein nach außen dichter Übergang vom Innern des Behälters 11 in die Kanüle 32 erreicht. Der Ansatz 60 sitzt klemmend auf dem Behälterauslaß.

## Patentansprüche

1. Applikationsvorrichtung für medizinische Flüssigkeiten, mit einem geschlossenen, mindestens teilweise flexiblen einstückigen Behälter (10), der ein zum Freilegen einer Behälteröffnung (20) abtrennbares Teil (17) aufweist, einer Übertragungsvorrichtung aus einem Konnektor (22), welcher abdichtend an der freigelegten Behälteröffnung (20) anbringbar ist, und einer an dem Konnektor (20) vorgesehenen oder mit diesem verbindbaren Kanüle (30;30a).

2. Applikationsvorrichtung nach Anspruch 1, dadurch gekennzeichnet, daß der Behälter (10) eine aus Kunststoff bestehende Ampulle ist.

3. Applikationsvorrichtung nach Anspruch 1 oder 2, dadurch gekennzeichnet, daß der Konnektor (22) an seinem behälterseitigen Ende eine Muffe (25) mit mindestens einer nach innen vorstehenden Dichtrippe (27) aufweist.

4. Applikationsvorrichtung nach einem der Ansprüche 1-3, dadurch gekennzeichnet, daß der Konnektor (22) einen in die Behälteröffnung (20) eindringenden Ansatz (23) aufweist, der sich zum Behälter (10) hin verjüngt und dessen dicker Bereich (23b) eine die Behälteröffnung (20) abdichtende Dichtung bildet.

5. Applikationsvorrichtung nach einem der Ansprüche 1-4, dadurch gekennzeichnet, daß der Konnektor (22b,22c,22d) einen in die Behälteröffnung (20) eindringenden zentrierenden Ansatz (23) und einen die Behälteröffnung (20) übergreifenden Dichtkragen (37) aufweist.

6. Applikationsvorrichtung nach Anspruch 5, dadurch gekennzeichnet, daß der Dichtkragen (37) dem Konnektor (22b,22d) einstückig angeformt ist.

7. Applikationsvorrichtung nach Anspruch 5, dadurch gekennzeichnet, daß der Dichtkragen (37) aus einem separaten Dichtungsring (43) besteht.

8. Applikationsvorrichtung nach Anspruch 4 oder 5, dadurch gekennzeichnet, daß der Ansatz (23) oder die Muffe (25) ein Schneidgewinde (42) aufweist.

9. Applikationsvorrichtung nach einem der Ansprüche 1-8, dadurch gekennzeichnet, daß der Konnektor (22b,22d) an seinem kanülenseitigen Ende eine Muffe (38) mit einer elastischen Innenrippe (39) aufweist, welche den Wulst (51) eines in die Muffe (38) eingeführten Kanülenansatzes (50) rastend hintergreift.

10. Applikationsvorrichtung nach einem der Ansprüche 1-7, dadurch gekennzeichnet, daß der Konnektor an seinem kanülenseitigen Ende eine Muffe zum Einführen eines Kanülenansatzes (50) aufweist, wobei der Kanülenansatz in der Muffe mit einem Luer-Lock-Verschluß verriegelbar ist.

11. Applikationsvorrichtung nach einem der Ansprüche 1-10, dadurch gekennzeichnet, daß der Konnektor (22e) an der Behälterseite einen Ansatz (23) in Form eines männlichen Luer-Konus aufweist.

12. Applikationsvorrichtung nach einem der Ansprüche 1-10, dadurch gekennzeichnet, daß der Konnektor ganz oder teilweise aus Elastomermaterial besteht.

13. Applikationsvorrichtung nach einem der Ansprüche 1-12, dadurch gekennzeichnet, daß der Behälter (10) eine von der Seite der Behälteröffnung (20) ausgehende Graduierung (19) trägt.

14. Applikationsvorrichtung nach einem der vorhergehenden Ansprüche, dadurch gekennzeichnet, daß der Konnektor (22f) einen hohlen Ansatz (60) aufweist, in dem ein Dichtelement (61) angeordnet ist, an dem der Rand der Behälteröffnung (20) abdichtend anliegt.

15. Konnektor für eine Applikationsvorrichtung nach einem der Ansprüche 1-14.
